(19) Europäisches Patentamt
European Patent Office
Office européen des brevets

(11) Veröffentlichungsnummer: **0 000 113**
**B1**

(12) # EUROPÄISCHE PATENTSCHRIFT

(45) Veröffentlichungstag der Patentschrift: **16.09.81**

(51) Int. Cl.³: **C 07 D 237/04, A 61 K 31/50**

(21) Anmeldenummer: **78100001.3**

(22) Anmeldetag: **01.06.78**

(54) **Dihydropyridazone, ihre Herstellung und sie enthaltende Arzneimittel sowie Dihydropyridazone zur Verwendung für therapeutische Behandlung.**

(30) Priorität: **18.06.77 DE 2727481**

(43) Veröffentlichungstag der Anmeldung:
**10.01.79 Patentblatt 79/1**

(45) Bekanntmachung des Hinweises auf die Patenterteilung:
**16.09.81 Patentblatt 81/37**

(84) Benannte Vertragsstaaten:
**BE CH DE FR GB NL**

(56) Entgegenhaltungen:
**DE - A - 1 670 158**
**DE - A - 2 123 246**
**DE - A - 2 150 436**
**DE - A - 2 304 977**

(73) Patentinhaber: **BASF Aktiengesellschaft**
**Carl-Bosch-Strasse 38**
**D-6700 Ludwigshafen (DE)**

(72) Erfinder: **Lebkuecher, Rolf, Dr.**
**Kranichstrasse 9**
**D-6700 Ludwigshafen (DE)**
Erfinder: **Thyes, Marco, Dr.**
**Mundenheimer Strasse 148**
**D-6700 Ludwigshafen (DE)**
Erfinder: **Koenig, Horst, Dr.**
**Pariser Strasse 4**
**D-6700 Ludwigshafen (DE)**
Erfinder: **Lehmann, Hans Dieter, Dr.**
**Im Hefen 15**
**D-6945 Hirschberg-Leuterhausen (DE)**
Erfinder: **Gries, Josef, Dr.**
**Roemerweg 43**
**D-6706 Wachenheim (DE)**
Erfinder: **Lenke, Dieter, Dr.**
**Kekuléplatz 1**
**D-6700 Ludwigshafen (DE)**
Erfinder: **Kunze, Johannes, Dr.**
**Hoferweg 4**
**D-8350 Plattling (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 113 B1

# Dihydropyridazone, ihre Herstellung und sie enthaltende Arzneimittel sowie Dihydropyridazone zur Verwendung für therapeutische Behandlung

Die Erfindung betrifft 6-(p-Acylaminophenyl)-4,5-dihydropyridazone-(3), ihre Herstellung und pharmazeutische Zubereitungen, die 6-(p-Acylaminophenyl)-4,5-dihydropyridazone-(3) enthalten.

Es wurde gefunden, daß im Acylrest durch ein Halogenatom substituierte 6-(p-Acylaminophenyl)-4,5-dihydropyridazone-(3) der Formel I

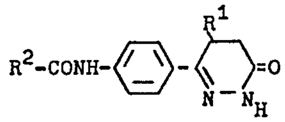

I,

in der für $R^1$ Wasserstoff oder ein Alkylrest mit 1 bis 3 C-Atomen steht und $R^2$, wenn für $R^1$ ein Wasserstoffatom steht, einen durch ein Halogenatom substituierten Alkylrest mit 3 bis 6 C-Atomen oder einen $\beta$-Halogenäthylrest oder, wenn für $R^1$ ein Alkylrest mit 1 bis 3 C-Atomen steht, einen durch ein Halogenatom substituierten Alkylrest mit 1 bis 6 C-Atomen bedeutet, wertvolle pharmakologische Eigenschaften aufweisen.

Alkylreste für $R^1$ sind insbesondere Methyl, Äthyl und Propyl. Durch Halogen substituierte Alkylreste für $R^2$, wenn für $R^1$ ein Wasserstoffatom steht, sind beispielsweise 2-Chloräthyl, 2-Bromäthyl, 2-Fluoräthyl, 2-Jodäthyl, 1-Chlorpropyl, 1-Brompropyl, 1-Fluorpropyl, 1-Jodpropyl, 2-Chlorpropyl, 3-Chlorpropyl, 3-Brompropyl, 3-Fluorpropyl, 3-Jodpropyl, 1-Chlorisopropyl, 1-Bromisopropyl, 1-Jodisopropyl, 2-Chlorisopropyl, 2-Bromisopropyl, 1-Chlorbutyl, 1-Brombutyl, 1-Fluorbutyl, 4-Chlorbutyl, 4-Brombutyl, 4-Fluorbutyl, 1-Chlorisobutyl, 1-Bromisobutyl, 2-Chlorisobutyl, 1-Chlor-sec-butyl, 1-Brom-sec-butyl, 3-Chlor-sec-butyl, Chlortertiärbutyl, Bromtertiärbutyl, 1-Chloramyl, 1-Bromamyl, 5-Bromamyl, 1-Äthyl-1-chlorpropyl, 1-Äthyl-1-brompropyl.

Für den Fall, daß für $R^1$ ein Alkylrest steht, kommen für $R^2$ neben den oben genannten Bedeutungen noch beispielsweise Chlormethyl, Brommethyl, Fluormethyl, Jodmethyl, 1-Chloräthyl, 1-Bromäthyl, 1-Fluoräthyl oder 1-Jodäthyl in Betracht.

Die bevorzugten Verbindungen sind solche, in denen $R^1$ Wasserstoff oder Methyl und $R^2$, wenn $R^1$ Wasserstoff ist, einen durch ein Halogenatom, insbesondere ein Chlor- oder Bromatom, substituierten Alkylrest mit 3 oder 4 C-Atomen oder einen $\beta$-Halogenäthylrest, oder, wenn $R^1$ Methyl ist, einen durch ein Halogenatom, insbesondere ein Chlor- oder Bromatom, substituierten Alkylrest mit 1 bis 4 C-Atomen bedeutet.

Die Verbindungen der Formel I können hergestellt werden, indem man eine Verbindung der Formel II

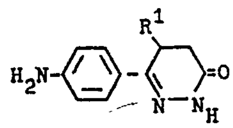

II,

in der $R^1$ die für Formel I angegebenen Bedeutungen hat, mit einem Acylierungsmittel der Formel III

$$R^2COY \qquad III,$$

in der $R^2$ die für Formel I angegebenen Bedeutungen hat und für Y OH, Chlor, Brom, ein niederer Alkoxyrest oder $OCOR^2$ steht, in an sich bekannter Weise umsetzt.

Gemäß den für Y angegebenen Bedeutungen sind zweckmäßige Acylierungsmittel die entsprechenden Carbonsäuren, Carbonsäurehalogenide, insbesondere Chloride und Bromide, Carbonsäureester, insbesondere Methyl- und Äthylester, und die entsprechenden Carbonsäureanhydride.

Die Acylierung wird unter an sich üblichen Bedingungen durchgeführt. In der Regel unter Verwendung von wenigstens einer äquimolaren Menge des Acylierungsmittels, zweckmäßig in Gegenwart eines Lösungsmittels und gegebenenfalls in Gegenwart einer Hilfsbase bei Temperaturen zwischen 0°C und 160°C, gegebenenfalls bei den Siedetemperaturen des Reaktionsgemisches und gegebenenfalls unter Anwendung von Druck.

Als Lösungsmittel kommen unter den Reaktionsbedingungen inerte Lösungsmittel, wie aromatische Kohlenwasserstoffe, beispielsweise Benzol oder Toluol, cyclische aliphatische Äther, wie Dioxan, oder Dialkylformamide, wie Dimethylformamid, in Betracht. Hilfsbasen als säurebindende Mittel sind zweckmäßigerweise anorganische Basen, wie Natrium- oder Kaliumcarbonat, Natrium- oder Kalium-hydrogencarbonat, oder tertiäre organische Amine, wie Triäthylamin.

Die Herstellung der 6-(p-Acylaminophenyl)-4,5-dihydropyridazone-(3) der Formel I wird durch das folgende Formelschema veranschaulicht:

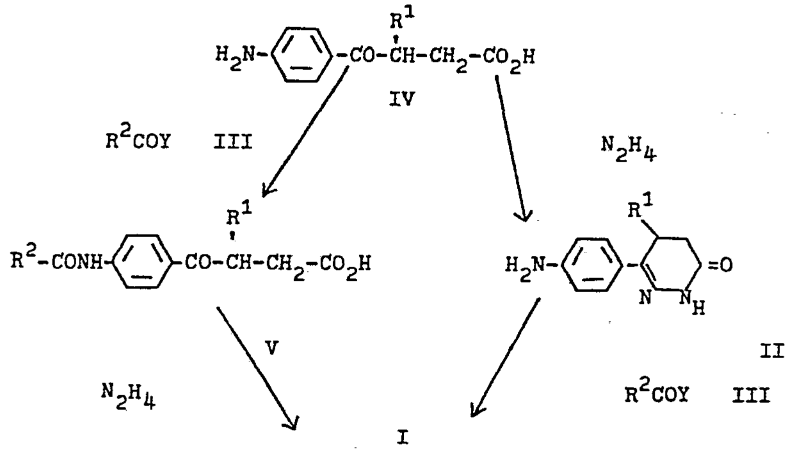


Demnach werden gemäß einer weiteren Ausführungsform die Verbindungen der Formel I hergestellt, indem man eine Verbindung der Formel IV, in der $R^1$ die für Formel I angegebenen Bedeutungen hat, mit einem Acylierungsmittel der Formel III

$$R^2COY \qquad III,$$

in der $R^2$ und Y die oben angegebenen Bedeutungen haben, unter den oben angegebenen Bedingungen acyliert und die erhaltene Acylverbindung V

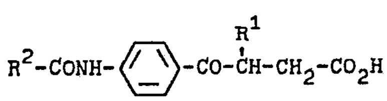
V,

mit Hydrazin cyclisiert.

Diese Ringschlußreaktion mit Hydrazin oder Hydrazinhydrat wird vorteilhaft mit einer äquimolekularen Menge Hydrazin in einem Lösungsmittel, insbesondere einem niederen Alkohol, wie Methanol, Äthanol oder Propanol, einem cyclischen Äther, wie Dioxan, oder einem Dialkylformamid, wie Dimethylformamid, bei Temperaturen von 60 bis 150°C, vorzugsweise 80 bis 120°C, durchgeführt.

Die als Ausgangssubstanzen verwendeten Verbindungen der Formel II und auch der Formel IV sind bekannt oder können unter den beispielsweise in den DT—OS 16 70 158 und 21 50 436 oder den US—PS 3 824 271 und 3 888 901 beschriebenen Bedingungen hergestellt werden.

Es wird darauf hingewiesen, daß die Verbindungen der Formel I in denen $R^1$ ungleich Wasserstoff ist, ein asymmetrisches C-Atom in 5-Stellung aufweisen und als Racemate vorliegen. Die vorliegende Erfindung soll die Enantiomeren mit einschließen. Falls eine Trennung gewünscht wird, wird diese zweckmäßigerweise auf der Stufe einer Verbindung der Formel II nach an sich üblichen Verfahren mit einer optisch aktiven Säure, wie Dibenzoylweinsäure oder Campher-10-sulfonsäure, über die Bildung diastereomerer Salze durchgeführt.

Nach den genannten Verfahren werden neben den in den Ausführungsbeispielen angegebenen Verbindungen beispielsweise erhalten:

6-(p-Bromoacetylaminophenyl)-4,5-dihydropyridazon-(3);
6-(p-Bromacetylaminophenyl)-5-methyl-4,5-dihydropyridazon-(3);
6-(p-Fluoracetylaminophenyl)-5-methyl-4,5-dihydropyridazon-(3);
6-(p-Jodacetylaminophenyl)-5-methyl-4,5-dihydropyridazon-(3);
6-[p-(3-Brompropionylamino)-phenyl]-5-methyl-4,5-dihydropyridazon-(3);
6-[p-(3-Fluorpropionylamino)-phenyl]-5-methyl-4,5-dihydropyridazon-(3);
6-[p-(3-Jodpropionylamino)-phenyl]-5-methyl-4,5-dihydropyridazon-(3);
6-[p-(2-Chlorbutyrylamino)-phenyl]-4,5-dihydropyridazon-(3);
6-[p-(2-Chlorbutyrylamino)-phenyl]-5-methyl-4,5-dihydropyridazon-(3);
6-[p-(3-Chlorbutyrylamino)-phenyl]-5-methyl-4,5-dihydropyridazon-(3);
6-[p-(2-Bromvalerylamino)-phenyl]-5-methyl-4,5-dihydropyridazon-(3);
6-[p-(2-Bromisovalerylamino)-phenyl]-5-methyl-4,5-dihydropyridazon-(3);
6-[p-(2-Äthyl-2-brombutyrylamino)-phenyl]-5-methyl-4,5-dihydropyridazon-(3);

Weiterhin wurde gefunden, daß die neuen Verbindungen der Formel I und darüber hinaus auch Verbindungen der Formel I, in denen, wenn $R^1$ Wasserstoff ist, $R^2$ einen Halogenmethyl- oder einen $\alpha$-Halogenäthylrest bedeutet, sich durch eine starke thrombozytenaggregationshemmende Wirkung und durch eine blutdrucksenkende Wirkung auszeichnen. Sie sind als Antihypertensiva und zur Prophylaxe und Therapie thromboembolischer Erkrankungen geeignet.

Zur Untersuchung der pharmakodynamischen Eigenschaften der erfindungsgemäßen Produkte wurden folgende Methoden verwendet:

1. Hemmung der durch Collagen induzierten Aggregation von Thrombozyten des Menschen in vitro

Thrombozytenreiches Plasma wird durch Zentrifugation (300 g, 10 min Dauer bei 4°C) von venösem Citratblut gewonnen. Die photometrische Messung der Thrombozytenaggregation erfolgt unter Zusatz von $MgCl_2$ (Endkonzentration 10 mmol/l) und von Collagen Stago (Endkonzentration 0,02 mg/ml) im Born-Aggregometer Mk 3. Als Aggregationsmaß findet die maximale Extinktionsänderung/sec Verwendung.

Die Prüfung der aggregationshemmenden Wirksamkeit der Substanzen wird nach einer Inkubationszeit von 10 min vorgenommen.

Als EC 50% wird die Konzentration bestimmt, welche eine 50%ige Hemmung der Aggregation verursacht.

2. Hemmung der durch Collagen induzierten Aggregation von Thrombozyten der Ratte ex vivo

Die Substanzen werden Gruppen von 10—15 männlichen Sprague- Dawley-Ratten (200—250 g) oral appliziert. 2—4 Stunden nach der Applikation wird in Aether-Narkose Blut entnommen und durch Zentrifugation thrombozytenreiches Plasma gewonnen. Die Messung der Aggregation nach Collagen erfolgt wie oben angegeben.

Als ED 33% wird die Dosis bestimmt, welche die durch Collagen induzierte Thrombozytenaggregation um 33% hemmt.

3. Blutdrucksenkende Wirkung an der narkotisierten Ratte

Zur Testung der blutdrucksenkenden Wirkung erhalten Gruppen von 3—5 männlichen Sprague-Dawley-Ratten (240—280 g) in Urethan-Narkose (1,78 mg/kg i.p.) die Substanzen intraperitoneal appliziert. Die Messung des Blutdrucks in der A.carotis erfolgt über Statham-Transducer. Als ED 20% wird die Dosis bestimmt, welche den mittleren Carotisblutdruck um 20% senkt.

4. Antihypertensive Wirkung an spontan hypertonen Ratten

Gruppen von 4—8 männlichen spontan hypertonen Okamoto- Ratten (270—340 g) werden die Substanzen oral appliziert.

Vor und 2 Stunden nach der Applikation wird der systolische Blutdruck unblutig mit Hilfe von Piezokristallaufnehmern gemessen.

Als ED 20% wird unter Berücksichtigung der Werte unbehandelter Kontrolltiere die Dosis bestimmt, welche den systolischen Druck um 20% senkt.

Die Berechnung der wirksamen Dosen bzw. Konzentrationen erfolgte aus den linearen Beziehungen zwischen den Logarithmen der Dosen bzw. Konzentrationen und der Wirkung mit Hilfe der Regressionsanalyse.

Als Referenzsubstanz für die Hemmung der Thrombozytenaggregation diente Acetylsalicylsäure, für die blutdrucksenkende Wirkung Dihydralazin.

Tabelle 1

| Beisp.-Nr. | Thrombozytenaggregationshemmung[2)] EC 50% | R.W. | Blutdrucksenkung[1)] ED 20% | R.W. |
|---|---|---|---|---|
| 1 | 0,25 | 1 980 | 0,37 | 0,92 |
| 3 | 0,11 | 4 490 | 0,15 | 2,27 |
| 4 | 0,27 | 1 830 | 0,53 | 0,64 |
| 5 | 0,031 | 15 900 | 0,0079 | 43,04 |
| 6 | 0,35 | 1 410 | 10 | 0,03 |
| 7 | 0,30 | 1 650 | 0,045 | 7,56 |
| 8 | 1,70 | 291 | 0,32 | 1,06 |
| 9 | 0,27 | 1 830 | 1,49 | 0,23 |
| 11 | 0,47 | 1 050 | 0,61 | 0,56 |
| 12 | 0,44 | 1 120 | 0,34 | 1,00 |
| 16 | 0,58 | 852 | 0,40 | 0,85 |
| 17 | 0,89 | 555 | 2,93 | 0,12 |
| Dihydralazin | 149 | 3,32 | 0,34 | 1,00 |
| Acetylsalicylsäure | 494 | 1,00 | — | — |

1) Ratte. Urethan-Narkose. Appl. i.p. ED 20% [mg/kg] = Dosis, welche den Blutdruck um 20% senkt. R.W. = Relative Wirksamkeit; Dihydralazin = 1,00.

2) Thrombozyten (Mensch) in vitro. EC 50% [mg/l] = Konzentration, welche die durch Collagen induzierte Aggregation um 50% hemmt. R.W. = Relative Wirksamkeit; Acetylsalicylsäure = 1,00.

Tabelle 2

| Substanz | Hemmung der Thrombozytenaggregation 1) ED 33% | R.W. | Antihypertensive Wirkung 2) ED 20% | R.W. | Toxizität 3) LD 50 |
|---|---|---|---|---|---|
| Beisp. 5 | 0,82 | 163 | 1,16 | 5,9 | 231 |
| Acetylsalicylsäure | 134 | 1,0 | — | — | 167 |
| Dihydralazin | — | — | 6,85 | 1,0 | 206 |

1) Ratte. Appl. per os. Entsprechend der maximalen Wirksamkeit wurde Beispiel 5 2 Stunden, Acetylsalicylsäure 4 h vor Messung der Aggregation appliziert. ED 33% [mg/kg] = Dosis, welche die durch Collagen induzierte Aggregation um 33% hemmt. R.W. = Relative Wirksamkeit.

2) Spontan hypertone Ratte. Appl. per os. ED 20% [mg/kg] = Dosis, welche den Blutdruck im Vergleich zur Kontrollgruppe um 20% senkt.

3) Maus. Appl. i.p.

Die Ergebnisse (Tabelle 1) zeigen für die erfindungsgemäßen Verbindungen eine außerordentlich starke Hemmung der durch Collagen induzierten Aggregation von Thrombozyten des Menschen. Die Wirkung ist 291—15 900mal stärker als die des bekannten aggregationshemmenden Pharmakons Acetylsalicylsäure.

Außer der Hemmung der Thrombozytenaggregation tritt eine blutdrucksenkende Wirkung von unterschiedlicher Stärke auf. Die Verbindungen Beispiele Nr. 5 und Nr. 7 wirken 43 bzw. 7,6mal stärker blutdrucksenkend als das bekannte Antihypertonikum Dihydralazin. Eine Reihe weiterer Verbindungen (Beispiele 1, 3, 4, 8, 12 und 16) sind etwa ebenso aktiv wie Dihydralazin. Bei Verbindung 9 und 17, besonders aber bei Beispiel 6 ist die blutdrucksenkende Wirkung gering. Hier liegt eine hohe Spezifität der thrombozytenaggregationshemmenden Wirkung vor. Pharmakotherapeutisch sind Verbindungen mit thrombozytenaggregationshemmender und blutdrucksenkender Wirkung ebenso wünschenswert wie solche die thrombozytenaggregationshemmend wirken und den Blutdruck nur wenig beeinflussen.

An der Verbindung des Beispiels 5 (Tab. 2) wird nachgewiesen, daß die sehr starke Hemmung der Thrombozytenaggregation auch nach oraler Applikation festzustellen ist. Die Substanz senkt außerdem in der niedrigen oralen Dosis von 1,2 mg/kg den Blutdruck spontan hypertensiver Ratten um 20%. Die akute Toxizität von Beispiel 5 ist etwas geringer als die von Acetylsalicylsäure und Dihydralazin.

Gegenstand der vorliegenden Erfindung sind demnach auch therapeutische Mittel oder Zubereitungen, die neben üblichen Träger-und Verdünnungsmitteln die neuen Verbindungen der Formel I als Wirkstoff enthalten, sowie diese Verbindungen oder auch Verbindungen der Formel I, in denen, wenn $R^1$ Wasserstoff ist, $R^2$ einen Halogenmethyl oder einen $\alpha$-Halogenäthylrest bedeutet, zur Anwendung als Antihypertensiva und zur Prophylaxe und Therapie thromboembolischer Erkrankungen.

Es sei erwähnt, daß die Verbindungen 6-(p-Chloracetylaminophenyl)-4,5-dihydropyridazon-(3) und 6-[p-(2-Chlorpropionylamino)-phenyl]-4,5-dihydropyridazon-(3) in der DT—OS 21 23 246 als Zwischenprodukte beschrieben werden. Für Acylaminophenyl-dihydropyridazone, die im Acyl nicht durch Halogen substituiert sind, sind entzündungshemmende und blutdrucksenkende Eigenschaften beschrieben (vgl. DT—OS 16 70 158 und DT—OS 21 50 436). Die Hemmung der Thrombozytenaggregation durch die erfindungsgemäß zu verwendenden Verbindungen ist eine völlig unerwartete Wirkung.

Die therapeutischen Mittel oder Zubereitungen werden mit den üblichen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Dabei kommen beim Menschen Dosen von 1 bis 100 mg in Betracht, wobei die orale Applikation bevorzugt ist.

Darreichungsformen, die zur oralen Applikation geeignet sind, sind beispielsweise Tabletten, Filmtabletten, Dragees, Kapseln, Pillen, Pulver, Lösungen, Suspensionen oder Depotformen.

Zur praktischen Anwendung werden die erfindungsgemäß zu verwendenden Verbindungen mit den in der galenischen Pharmazie üblichen Trägerstoffen verarbeitet. Die entsprechenden Tabletten können beispielsweise durch Mischen des Wirkstoffs mit bekannten Hilfsstoffen, beispielsweise inerten Verdünnungsmitteln, wie Dextrose, Zucker, Sorbit, Polyvinylpyrrolidon, Mannit, Calciumcarbonat, Calciumphosphat oder Milchzucker, Sprengmitteln, wie Maisstärke, Alginsäure oder Polyvinylpyrrolidon, Bindemitteln wie Stärke oder Gelatine, Gleitmitteln, wie Magnesiumstearat oder Talkum, und/oder Mitteln zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Die Herstellung der erfindungsgemäß als Arzneimittel zu verwendenden 6-(p-Acylaminophenyl)-4,5-dihydropyridazone-(3) wird durch die folgenden Beispiele näher erläutert.

## Beispiel 1

30,2 g (0,16 Mol) 6-(p-Aminophenyl)-4,5-dihydropyridazon-(3) werden mit 18,1 g (0,16 Mol) Chloracetylchlorid und 150 ml absolutem Benzol 2 Stunden unter Rückfluß gekocht. Man saugt bei Raumtemperatur ab, wäscht zuerst mit Benzol, dann mit Wasser und trocknet im Vakuum bei 80°C. Man erhält 34,6 g (81% d.Th.) 6-(p-Chloracetylaminophenyl)-4,5-dihydropyridazon-(3), beiger Festkörper, Schmelzpunkt 233°C (Zers.) (umkristallisiert aus Dimethylformamid/Wasser).

Analyse für $C_{12}H_{12}ClN_3O_2$:
ber.: C 54,2 H 4,5 Cl 13,3 N 15,8 O 12,0%
gef.: C 54,1 H 4,5 Cl 13,6 N 15,8 O 12,4%

## Beispiel 2

a) 20 g (103 mMol) $\beta$-(p-Aminobenzoyl)-propionsäure und 12,9 g (114 mMol) Chloracetylchlorid

werden mit 200 ml absolutem Toluol 4 Stunden bei 80°C gehalten. Man saugt bei 10°C ab, wäscht mit Wasser und trocknet im Vakuum bei 50°C. Man isoliert 25,1 g (90% d.Th.) β-(p-Chloracetylaminobenzoyl)-propionsäure, hellbraune Kristalle, Schmelzpunkt 184—185°C (umkristallisiert aus Aceton).

Analyse für $C_{12}H_{12}ClNO_4$:
ber.: C 53,4 H 4,5 Cl 13,1 N 5,2%
gef.: C 53,6 H 4,6 Cl 13,0 N 5,2%

b) 4,0 g (14,8 mMol) β-(p-Chloracetylaminobenzoyl)-propionsäure werden mit 0,74 g (14,8 mMol) Hydrazinhydrat und 70 ml Äthanol 3 Stunden am Rückfluß gehalten. Nach dem Absaugen bei 10°C und dem Trocknen im Vakuum bei 50°C erhält man 3,3 g (84% d.Th.) 6-(p-Chloracetylaminophenyl)-4,5-dihydropyridazon-(3), hellgelbe Kristalle (identisch mit der Verbindung aus Beispiel 1; Schmelzpunkt, IR, NMR).

Beispiel 3

6,4 g (31,5 mMol) 6-(p-Aminophenyl)-5-methyl-4,5-dihydropyridazon-(3) und 4,2 g (37,1 mMol) Chloracetylchlorid werden in 150 ml absolutem Benzol 4 Stunden am Rückfluß gehalten. Man saugt bei 0°C ab, wäscht mit Wasser und kristallisiert aus Äthanol/Wasser um. Man erhält 3,8 g (43% d.Th.) 6-(p-Chloracetylaminophenyl)-5-methyl-4,5-dihydropyridazon-(3), hellgelbe Kristalle, Schmelzpunkt 235,5 bis 236,5°C.

Analyse für $C_{13}H_{14}ClN_3O_2$:
ber.: C 55,8 H 5,0 Cl 12,7 N 15,0 O 11,4%
gef.: C 55,8 H 5,1 Cl 12,4 N 15,2 O 11,9%

Beispiel 4

47,2 g (0,25 Mol) 6-(p-Aminophenyl)-4,5-dihydropyridazon-(3) werden mit 35,6 g (0,28 Mol) 2-Chlorpropionylchlorid und 250 ml absolutem Benzol 2 Stunden unter Rückfluß gekocht. Man saugt bei 10°C ab, wäscht zuerst mit Benzol, dann mit Wasser und trocknet im Vakuum bei 100°C. Man erhält 64,8 g (93% d.Th.) 6-[p-(2-Chlorpropionylamino)-phenyl]-4,5-dihydropyridazon-(3), beiger Festkörper, Schmelzpunkt 243 bis 244°C (Zers.) (umkristallisiert aus Propanol).

Analyse für $C_{13}H_{14}ClN_3O_2$:
ber.: C 55,8 H 5,0 Cl 12,7 N 15,0 O 11,4%
gef.: C 55,2 H 4,9 Cl 13,1 N 14,9 O 11,9%

Beispiel 5

6,0 g (29,6 mMol) 6-(p-Aminophenyl)-5-methyl-4,5-dihydropyridazon-(3) und 4,1 g (32,3 mMol) 2-Chlorpropionylchlorid werden mit 100 ml absolutem Toluol 4 Stunden bei 80°C gehalten. Man saugt bei 10°C ab, wäscht mit Wasser und trocknet im Vakuum bei 50°C. Man isoliert 7,9 g (91% d.Th.) 6-[p-(2-Chlorpropionylamino)-phenyl]-5-methyl-4,5-dihydropyridazon-(3), beige Kristalle, Schmelzpunkt 215 bis 217°C (umkristallisiert aus Methanol).

Analyse für $C_{14}H_{16}ClN_3O_2$:
ber.: C 57,2 H 5,5 Cl 12,1 N 14,3 O 10,9%
gef.: C 57,1 H 5,5 Cl 12,1 N 14,6 O 11,2%

Beispiel 6

18,9 g (0,10 Mol) 6-(p-Aminophenyl)-4,5-dihydropyridazon-(3) werden mit 15,2 g (0,12 Mol) 3-Chlorpropionylchlorid und 90 ml absolutem Benzol 2 Stunden am Rückfluß gehalten Man saugt bei 10°C ab, wäscht zuerst mit Benzol, dann mit Wasser und trocknet im Vakuum bei 100°C. Man erhält 27,2 g (97% d.Th.) 6-[p-(3-Chlorpropionylamino)-phenyl]-4,5-dihydropyridazon-(3), farblose Substanz, Schmelzpunkt 226 bis 227°C (Zers.) (umkristallisiert aus Dimethylformamid/Wasser).

Analyse für $C_{13}H_{14}ClN_3O_2$:
ber.: C 55,8 H 5,0 Cl 12,7 N 15,0 O 11,4%
gef.: C 56,0 H 5,3 Cl 12,7 N 15,0 O 11,4%

Beispiel 7

6,0 g (29,6 mMol) 6-(p-Aminophenyl)-5-methyl-4,5-dihydropyridazon-(3) und 4,1 g (32,3 mMol) 3-Chlorpropionylchlorid werden mit 100 ml absolutem Toluol 4 Stunden bei 80°C gerührt. Man saugt bei 10°C ab, wäscht mit Wasser und trocknet im Vakuum bei 50°C. Man erhält 5,8 g (67% d.Th.) 6-[p-(3-Chlorpropionylamino)-phenyl]-5-methyl-4,5-dihydropyridazon-(3), beige Kristalle, Schmelzpunkt 221 bis 223°C (Zers.) (umkristallisiert aus Methanol).

Analyse für $C_{14}H_{16}ClN_3O_2$:
ber.: C 57,2 H 5,5 Cl 12,1 N 14,3 O 10,9%
gef.: C 57,2 H 5,6 Cl 12,0 N 14,4 O 11,6%

Beispiel 8

6,0 g (29,6 mMol) 6-(p-Aminophenyl)-5-methyl-4,5-dihydropyridazon-(3) und 4,6 g (32,6 mMol)

4-Chlorbutyrylchlorid werden mit 100 ml absolutem Toluol 6 Stunden bei 80°C gehalten. Man saugt bei 10°C ab, wäscht mit Wasser und trocknet im Vakuum bei 50°C. Man erhält 8,5 g (93% d.Th.) 6-[p-(4-Chlorbutyrylamino)-phenyl]-5-methyl-4,5-dihydropyridazon-(3), beige Kristalle, Schmelzpunkt 176 bis 178°C (umkristallisiert aus Methanol).

Analyse für $C_{15}H_{18}ClN_3O_2$:
ber.: C 58,6 H 5,9 Cl 11,5 N 13,7 O 10,4%
gef.: C 58,4 H 5,9 Cl 11,4 N 13,8 O 10,9%

In der folgenden Tabelle sind weitere Ausführungsbeispiele zusammengestellt. Die Herstellung dieser Dihydropyridazone erfolgte nach der in Beispiel 7 beschriebenen Methode.

TABELLE

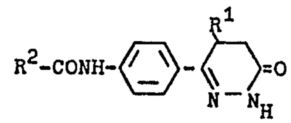

| Beispiel | $R^1$ | $R^2$ | Schmp. [°C] | Analyse (%) | | | | |
|---|---|---|---|---|---|---|---|---|
| | | | | | C | H | Hal | N |
| 9 | $-CH_3$ | $CH_3-CH_2-CH(Br)-$ | 213 – 214 (Zers.) | ber.: | 51,2 | 5,1 | 22,7 | 11,9 |
| | | | (DMF / Wasser) | gef.: | 51,3 | 5,1 | 22,3 | 12,2 |
| 10 | $-H$ | $CH_3-CH_2-CH(Br)-$ | 201 – 202 | ber.: | 49,7 | 4,8 | 23,6 | 12,4 |
| | | | (DMF / Wasser) | gef.: | 49,4 | 4,8 | 23,0 | 12,1 |
| 11 | $-CH_3$ | $Cl-CH_2-C(CH_3)_2-$ | 203 – 204 | ber.: | 59,7 | 6,3 | 11,0 | 13,1 |
| | | | (DMF / Wasser) | gef.: | 59,6 | 6,3 | 10,8 | 13,3 |
| 12 | $-CH_3$ | $Br-CH_2-C(CH_3)_2-$ | 189 – 190 | ber.: | 52,5 | 5,5 | 21,8 | 11,5 |
| | | | (DMF / Wasser) | gef.: | 52,6 | 5,8 | 21,2 | 11,9 |

| Beispiel | R¹ | R² | Schmp. [°C] | Analyse (%) | C | H | Hal | N |
|---|---|---|---|---|---|---|---|---|
| 13 | $-H$ | 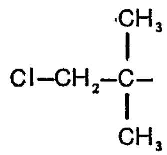 | 200 – 202 | ber.: | 58,5 | 5,9 | 11,5 | 13,7 |
| | | | (DMF /Wasser) | gef.: | 58,6 | 6,0 | 11,3 | 13,7 |
| 14 | $-H$ | 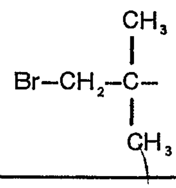 | 213 – 214 | ber.: | 51,2 | 5,1 | 22,7 | 11,9 |
| | | | (Propanol /Wasser) | gef.: | 51,5 | 5,2 | 21,9 | 12,1 |
| 15 | $-H$ | $Cl-CH_2-CH_2-CH_2-$ | 190 – 191 | ber.: | 57,2 | 5,5 | 12,1 | 14,3 |
| | | | (DMF /Wasser) | gef.: | 57,4 | 5,6 | 11,7 | 14,4 |
| 16 | $-CH_3$ | 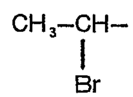 | 223 – 224 (Zers.) | ber.: | 49,7 | 4,8 | 23,6 | 12,4 |
| | | | (DMF /Wasser) | gef.: | 50,1 | 4,9 | 23,4 | 12,4 |
| 17 | $-CH_3$ | 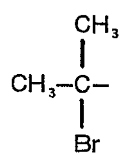 | 205 – 206 (Zers.) | ber.: | 51,2 | 5,1 | 22,7 | 11,9 |
| | | | (Propanol /Wasser) | gef.: | 51,1 | 5,4 | 21,9 | 12,2 |

Formulierungsbeispiele, die in üblicher Weise hergestellt werden:

1. Tabletten:

| | |
|---|---|
| Wirkstoff | 10 mg |
| Polyvinylpyrrolidon (mittl. M.G. 25 000) | 170 mg |
| Polyäthylenglykol (mittl. M.G. 4 000) | 14 mg |
| Hydroxypropylmethylcellulose | 40 mg |
| Talkum | 4 mg |
| Magnesiumstearat | 2 mg |
| | 240 mg |

Der Wirkstoff wird mit Polyvinylpyrrolidon in 10%iger wäßriger Lösung befeuchtet, durch ein Sieb mit der lichten Maschenweite 1,0 mm getrieben und bei 50°C getrocknet. Dieses Granulat wird mit Polyäthylenglykol (mittl. M.G. 4 000), Hydroxypropylmethylcellulose, Talkum und Magnesiumstearat vermischt und zu Tabletten à 240 mg verpreßt.

2. Beispiel für Dragees:

| | |
|---|---|
| Wirkstoff | 10 mg |
| Lactose | 90 mg |
| Maisstärke | 60 mg |
| Polyvinylpyrrolidon | 6 mg |
| Magnesiumstearat | 1 mg |
| | 167 mg |

Die Mischung der Wirkstoffsubstanz mit Lactose und Maisstärke wird mit einer 8%igen wäßrigen Lösung des Polyvinylpyrrolidons durch Sieb 1,5 mm granuliert, bei 50°C getrocknet und nochmals durch Sieb 1,0 mm getrieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

**Patentansprüche**

1. 6-(p-Acylaminophenyl)-4,5-dihydropyridazone-(3) der Formel I

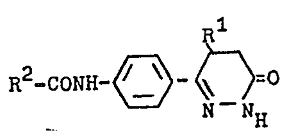

I,

in der für $R^1$ Wasserstoff oder ein Alkylrest mit 1 bis 3 C-Atomen steht und $R^2$, wenn für $R^1$ ein Wasserstoffatom steht, einen durch ein Halogenatom substituierten Alkylrest mit 3 bis 6 C-Atomen oder einen $\beta$-Halogenäthylrest oder, wenn für $R^1$ ein Alkylrest mit 1 bis 3 C-Atomen steht, einen durch ein Halogenatom substituierten Alkylrest mit 1 bis 6 C-Atomen bedeutet.

2. Verbindungen der Formel I, in denen $R^1$ Wasserstoff oder Methyl und $R^2$, wenn $R^1$ Wasserstoff ist, einen durch ein Halogenatom substituierten Alkylrest mit 3 oder 4 C-Atomen oder einen $\beta$-Halogenäthylrest oder, wenn $R^1$ Methyl ist, einen durch ein Halogenatom substituierten Alkylrest mit 1 bis 4 C-Atomen bedeutet.

3. 6-[p-(2-Chlorpropionylamino)-phenyl]-5-methyl-4,5-dihydropyridazon-(3).

4. Verfahren zur Herstellung von Verbindungen der Formel I nach Anspruch 1, dadurch gekennzeichnet, daß man eine Verbindung der Formel II

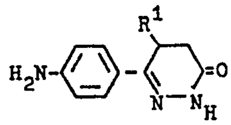

II,

in der $R^1$ die für Formel I angegebenen Bedeutungen hat, mit einem Acylierungsmittel der Formel III

$R^2COY$ III,

in der $R^2$ die für Formel I angegebenen Bedeutungen hat und für Y OH, Chlor, Brom, ein niederer Alkoxyrest oder $OCOR^2$ steht, gegebenenfalls in einem Lösungsmittel und gegebenenfalls in Gegenwart einer Base umsetzt.

5. Verfahren zur Herstellung von Verbindungen der Formel I nach Anspruch 1, dadurch gekennzeichnet, daß man eine Verbindung der Formel V

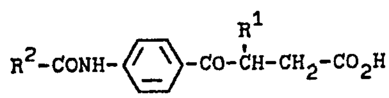

V,

in der $R^1$ und $R^2$ die für Formel I angegebenen Bedeutungen haben, mit Hydrazin cyclisiert.

6. Therapeutisches Mittel, gekennzeichnet durch einen Gehalt an einer Verbindung der Formel I nach Anspruch 1 als Wirkstoff neben üblichen Trägerstoffen und Verdünnungsmitteln.

7. Therapeutisches Mittel nach Anspruch 6, gekennzeichnet durch einen Gehalt an 6-[p-(2-Chlorpropionylamino)-phenyl]-5-methyl-4,5-dihydropyridazon-(3).

8. Eine Verbindung der Formel I nach Anspruch 1 oder eine Verbindung der Formel I, in der, wenn $R^1$ Wasserstoff ist, $R^2$ einen Halogenmethyl- oder einen $\alpha$-Halogenäthylrest bedeutet, zur Verwendung als Antihypertensivum und zur Prophylaxe und Therapie thrombo-embolischer Erkrankungen.

## Revendications

1. Dérivés de la 6-(p-acylaminophényl)-4,5-dihydro-pyridazone-(3) de la formule générale I

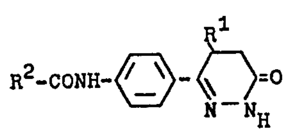

(I),

dans laquelle le symbole $R^1$ représente un atome d'hydrogène ou un radical alcoyle en $C_1$ à $C_3$ et le symbole $R^2$ désigne un radical alcoyle en $C_3$ à $C_6$ substitué par un atome d'halogène ou un radical $\beta$-halo-éthyle, lorsque $R^1$ représente un atome d'hydrogène, ou un radical alcoyle en $C_1$ à $C_6$ substitué par un atome d'halogène, lorsque $R^1$ représente un radical alcoyle en $C_1$ à $C_3$.

2. Dérivés de la formule générale I, dans laquelle $R^1$ représente un atome d'hydrogène ou un radical méthyle et $R^2$ un radical alcoyle en $C_3$ ou $C_4$ substitué par un atome d'halogène ou un radical $\beta$-halo-éthyle, si $R^1$ désigne un atome d'hydrogène, ou un radical alcoyle en $C_1$ à $C_4$ substitué par un atome d'halogène, lorsque $R^1$ désigne un radical méthyle.

3. La 6-[p-(2-chloropropionylamino)-phényl]-5-méthyl-4,5-dihydropyridazone-(3).

4. Procédé de préparation de dérivés de la formule générale I suivant la revendication 1, caractérisé en ce que l'on fait réagir un composé de la formule II

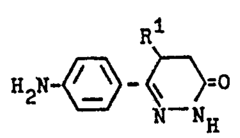

(II),

dans laquelle $R^1$ possède les significations définies pour la formule I, le cas échéant dans un solvant et éventuellement en présence d'une base, avec un agent d'acylation de la formule III

$R^2—COY$ (III),

dans laquelle $R^2$ possède les significations définies pour la formule I et Y désigne un radical —OH, un atome de chlore ou de brome, un groupe alcoxy inférieur ou un groupe $—OCOR^2$.

5. Procédé de préparation de dérivés de la formule générale I suivant la revendication 1, caractérisé en ce que l'on cyclise un composé de la formule V

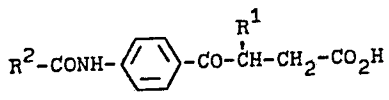

(V),

dans laquelle $R^1$ et $R^2$ possèdent les significations définies pour la formule I, par l'hydrazine.

6. Composition thérapeutique, caractérisée en ce qu'elle contient, à côté de véhicules et diluants usuels, un composé de la formule I suivant la revendication 1 comme principe actif.

7. Composition thérapeutique suivant la revendication 6, caractérisée en ce que le principe actif est la 6-[p-(2-chloropropionylamino)-phényl]-5-méthyl-4,5-dihydropyridazone-(3).

8. Composé de la formule I suivant la revendication 1 ou composé de la formule I, dans laquelle $R^1$ = H et $R^2$ représente un radical halo-méthyle ou $\alpha$-halo-éthyle, utilisé pour combattre l'hypertension et pour les traitements prophylactiques et thérapeutiques d'affections thrombo-emboliques.

## Claims

1. A 6-(p-acylaminophenyl)-4,5-dihydropyridaz-3-one of the formula I

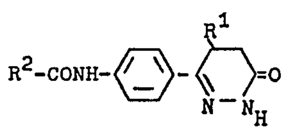

I

where $R^1$ is hydrogen or alkyl of 1 to 3 carbon atoms and $R^2$, if $R^1$ is hydrogen, is halogen-substituted alkyl of 3 to 6 carbon atoms or $\beta$-haloethyl or, if $R^1$ is alkyl of 1 to 3 carbon atoms, is halogen-subsituted alkyl of 1 to 6 carbon atoms.

2. A compound of the formula I, where $R^1$ is hydrogen or methyl and $R^2$, if $R^1$ is hydrogen, is halogen-substituted alkyl of 3 or 4 carbon atoms or $\beta$-haloethyl or, if $R^1$ is methyl, is halogen-substituted alkyl of 1 to 4 carbon atoms.

3. 6-[p-(2-Chloropropionylamino)-phenyl]-5-methyl-4,5-dihydropyridaz-3-one.

4. A process for the preparation of a compound of the formula I as claimed in claim 1, characterized in that a compound of the formula II

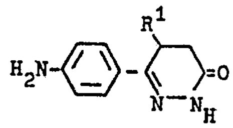

II

where $R^1$ has the meanings given for formula I, is reacted with an acylating agent of the formula III

$R^2COY$ III

where $R^2$ has the meanings given for formula I and Y is OH, chlorine, bromine, lower alkoxy or $OCOR^2$, in the presence or absence of a solvent and in the presence or absence of a base.

5. A process for the preparation of a compound of the formula I as claimed in claim 1, characterized in that a compound of the formula V

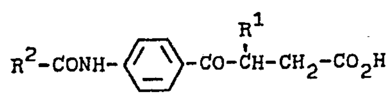

V

where $R^1$ and $R^2$ have the meanings given for formula I, is cyclized with hydrazine.

6. A therapeutic agent characterized by a content of a compound of the formula I as claimed in claim 1 as the active ingredient, in addition to conventional carriers and excipients.

7. A therapeutic agent as claimed in claim 6, characterized by a content of 6-[p-(2-chloropropionylamino)-phenyl]-5-methyl-4,5-dihydropyridaz-3-one.

8. A compound of the formula I as claimed in claim 1 or a compound of the formula I, in which, if $R^1$ is hydrogen, $R^2$ denotes halomethyl or $\alpha$-haloethyl, for use as an antihypertensive agent and for the prophylaxis and therapy of thromboembolic disorders.